# EUROPEAN PATENT APPLICATION

(11) **EP 2 036 521 A1**
(43) Date of publication of application: **18.03.2009**
(21) Application number: 07767622.9
(22) Date of filing: 26.06.2007
(51) Int. Cl.: A61F 13/15, A61F 13/472, A61F 13/53

(54) **ABSORBENT ARTICLE**

(30) Priority: 05.07.2006 JP 2006186118; 27.03.2007 JP 2007082500
(71) Applicant: KAO CORPORATION, Tokyo 103-8210 (JP)
(72) Inventor: NAKANO, Yukihiro, Haga-gun Tochigi 321-3497 (JP); KAWAGUCHI, Hiroko, Haga-gun Tochigi 321-3497 (JP); KASAI, Takao, Haga-gun Tochigi 321-3497 (JP)
(74) Representative: Vossius & Partner
(86) International application number: PCT/JP2007/062816
(87) International publication number: WO 2008/004463

(57) **Abstract**

An absorbent article 1 containing hydrophilic cellulosic fiber, which contains at least one surface active agent selected from the group consisting of a sugar alkyl ether and a sugar fatty acid ester, in a site except the skin-contacting surface 20 thereof. The site except the skin-contacting surface 20 is exemplified by an absorbent member 4 or an intermediate sheet 5. The hydrophilic cellulosic fiber may be present in a fiber aggregate 42 and/or a cover sheet 44 of the absorbent member 4. Another absorbent article of the invention includes a fiber layer containing more than 50% by weight of synthetic fiber. The synthetic fiber includes synthetic fiber treated with the surface active agent. The surface active agent is present in an amount more than 0.5 parts and not more than 12 parts by weight per 100 parts by weight of the total fiber content of the fiber layer.

## Description

### Field of the Invention

The present invention relates to an absorbent article.

### Background of the Invention

When using an absorbent article such as a sanitary napkin, passing back of a bodily fluid (e.g., menstrual blood) through under pressure sometimes occurs and it results rewet the wearer's skin to cause an unpleasant sticky feeling.
Such an unpleasant sticky feeling can be reduced by, for example, using a sheet with an uneven surface as a topsheet that is to come into direct contact with the skin thereby to reduce the skin contact area or a bulky nonwoven fabric as a topsheet.
In order to improve absorbency of an absorbent member of an absorbent article, studies have also been given to improvement of superabsorbent polymers used in the absorbent member, modification to the mode of the superabsorbent polymer being supported in the absorbent member, and improvement of the three-dimensional structure of the absorbent member.
However, demands for high quality absorbent articles have ever been increasing, and conventional techniques are likely to fail to fully meet future demands.

Patent Document 1 discloses a water-decomposable sheet comprising a water-decomposable base sheet coated on at least the skin-contacting side thereof with a mixture having at least two melting peak temperatures, reciting a sucrose ester as an example of a component of the mixture. Application of the mixture to the base sheet aims at improvement on soft and moist texture felt by the skin. The disclosure is silent on use of the water-decomposable sheet in a site except the skin-contacting side of an absorbent article.

Patent Document 2 teaches that a hydrophilic material obtained by adhering a surface active agent comprising a sugar alkyl ether to polyolefin nonwoven fabric or polyolefm perforated film exhibits long-lasting, durable hydrophilic properties and proposes using the hydrophilic material in absorbent articles. According to Patent Document 2, the preferred amount of the surface active agent to be adhered is 0.01% to 0.5% by weight based on the polyolefin nonwoven fabric or perforated film. It is mentioned that an increase in amount over 0.5% by weight results in little enhancement of the effect of the surface active agent.

Patent Document 1: JP 2005-290618A
Patent Document 2: JP 5-111509A

### Summary of the Invention

The present invention provides in its first aspect an absorbent article containing hydrophilic cellulosic fiber containing at least one surface active agent selected from the group consisting of a sugar alkyl ether and a sugar fatty acid ester.

The present invention provides in its second aspect an absorbent article including a fiber layer containing more than 50% by weight of synthetic fiber. The synthetic fiber includes synthetic fiber treated with at least one surface active agent selected from the group consisting of a sugar alkyl ether and a sugar fatty acid ester. The surface active agent is present in an amount more than 0.5 parts and not more than 12 parts by weight per 100 parts by weight of the total fiber content of the fiber layer.

The present invention provides in its third aspect an absorbent article containing a superabsorbent polymer and 1.5 to 15 parts by weight of at least one surface active agent selected from the group consisting of a sugar alkyl ether and a sugar fatty acid ester per 100 parts by weight of the superabsorbent polymer.

### Brief Description of the Drawings

Fig. 1 is a schematic fragmentary cross-sectional view of a sanitary napkin as an embodiment of the first aspect of the present invention.
Fig. 2 is a schematic fragmentary cross-sectional view of a sanitary napkin as an embodiment of the second aspect of the present invention.
Fig. 3 is a schematic fragmentary cross-sectional view of a sanitary napkin as an embodiment of the third aspect of the present invention.
Fig. 4(a) and Fig. 4(b) are each a plan view of an intermediate sheet (as a fiber sheet) having a surface active agent applied to parts thereof.

### Detailed Description of the Invention

The present invention will be described based on its preferred embodiments with reference to the accompanying drawings.

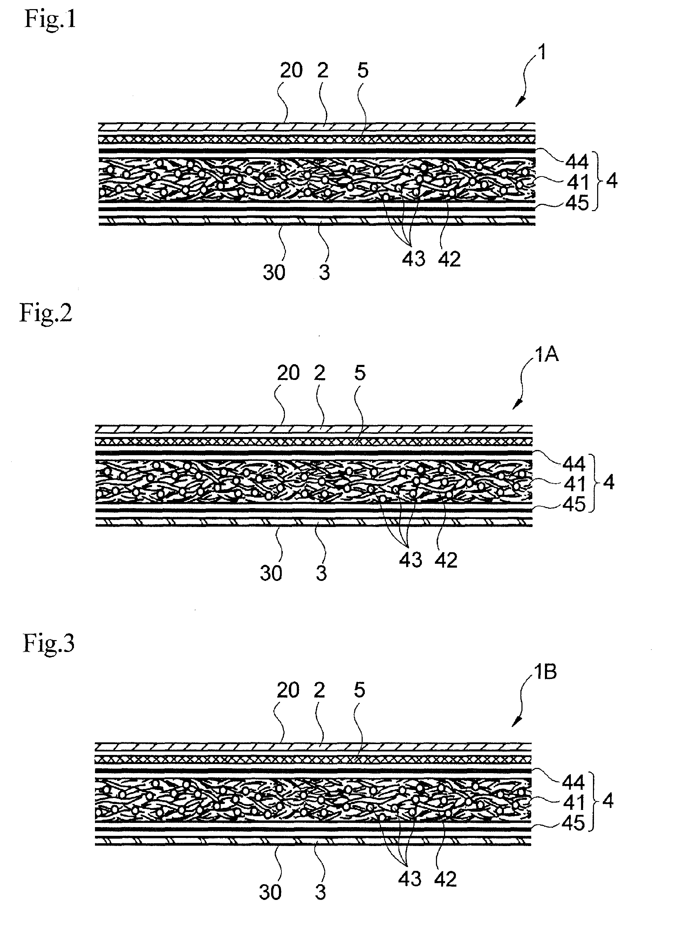

Fig. 1 is a schematic fragmentary cross-sectional view of a sanitary napkin 1 as a preferred embodiment of the absorbent article according to the first aspect of the present invention. As illustrated in Fig. 1, the sanitary napkin 1 includes a liquid permeable topsheet 2 defining the skin-contacting surface 20, a liquid impermeable or repellent leak-preventive sheet 3, an absorbent member 4 interposed between the sheets 2 and 3, and an intermediate sheet 5 interposed between the topsheet 2 and the absorbent member 4.
The absorbent member 4 includes an absorbent core 41, a cover sheet 44 covering the skin facing side of the absorbent core 41, and a cover sheet 45 covering the garment facing side of the absorbent core 41. The absorbent core 41 is composed of a fiber aggregate 42 and a superabsorbent polymer 43. The fiber aggregate 42 is made up of pulp fiber. The cover sheets 44 and 45 may be continuous with each other or independent of each other.

The napkin 1 contains hydrophilic cellulosic fiber containing at least one surface active agent selected from the group consisting of a sugar alkyl ether and a sugar fatty acid ester. The hydrophilic cellulosic fiber containing the surface active agent is present in a site other than the skin-contacting surface 20.
The skin-contacting surface 20 is a surface that is to be brought into contact with a wearer's skin while in use, and the garment-facing surface 30 is a surface opposite to the skin-contacting surface 20 and is to face a garment while in use.

The site except the skin-contacting surface is any position or site except the skin-contacting surface of a sheet defining the skin-contacting surface 20, which is the topsheet 2 in the present embodiment. In cases where the topsheet 2 has a multilayer structure, layers of the topsheet except a layer defining the skin-contacting surface 20 are included under the category of "a site except the skin-contacting surface". In cases where the topsheet 2 has a monolayer structure, the garment facing side thereof (opposite to the skin-contacting side) is included under the category of "a site except the skin-contacting surface". For example, when the topsheet 2 defining the skin-contacting surface 20 is a monolithic sheet containing hydrophilic cellulosic fiber and having only the garment facing side thereof (opposite to the skin-contacting side) coated with the surface active agent, the hydrophilic cellulosic fiber containing the surface active agent is said to be present at a site except the skin-contacting surface 20.
If the skin-contacting surface 20 is treated with the surface active agent, a body fluid would flow on the surface active agent-treated surface of the topsheet 2 rather than wick inside the absorbent member 4, which is likely to result in leakage.

The surface active agent that can be used in the invention is a sugar alkyl ether and/or a sugar fatty acid ester.
The sugar alkyl ether has a structure in which a sugar residual group and an alkyl group are single-bonded to an oxygen atom and is represented by formula: G-O-R₁, wherein G is a sugar residue, and R₁ is an alkyl group.
The sugar alkyl ethers that can be used in the invention include alkyl ethers of monosaccharides or polysaccharides (defined as two or more monosaccharide units condensed together). To ensure downward wicking properties, ethers between a monosaccharide or a polysaccharide composed of 2 to 30 monosaccharide units and an alkyl group having 1 to 60 carbon atoms are preferred, and alkyl glucosides (monosaccharide alkyl ethers) is more preferred.

In view of downward wicking properties and absorption rate, the alkyl moiety of the alkyl glucosides preferably has 1 to 22 carbon atoms, more preferably 4 to 18 carbon atoms, even more preferably 8 to 14 carbon atoms. The alkyl moiety may be either straight-chain or branched. While the alkyl moiety may have a chain length distribution, it is preferred that 60% by weight or more of alkyl chains have chain lengths within the above-recited range.

Alkyl etherification of all the five hydroxyl groups per glucose molecule being taken as 100% etherification, the alkyl glucoside is preferably a single compound or a mixture of compounds having a degree of etherification ranging from 2% to 60%. In view of absorption rate and downward wicking properties, the degree of etherification is more preferably 5% to 45%, even more preferably 15% to 35%.

The sugar fatty acid ester is a nonionic surface active agent in which a sugar and a saturated or unsaturated fatty acid are ester-bonded. The sugar fatty acid esters that can be used in the invention include monosaccharide fatty acid esters and polysaccharide (defined as two or more monosaccharide units condensed together) fatty acid esters. From the standpoint of downward wicking properties, esters between a monosaccharide or a polysaccharide having 2 to 30 monosaccharide units and a fatty acid having 1 to 60 carbon atoms are preferred, and sucrose (disaccharide) fatty acid esters is more preferred.

In view of downward wicking performance and absorption rate, the fatty acid moiety of the sucrose fatty acid esters, which is represented by formula: RCOOH, is preferably the one in which R is an alkyl or an alkenyl group each of which has 1 to 30 carbon atoms and may be substituted with a phenyl or a phenylene group, more preferably the one in which R is an alkyl or an alkenyl group each having 4 to 22 carbon atoms, even more preferably the one in which R is an alkyl or an alkenyl group each having 8 to 18 carbon atoms. The fatty acid may be straight-chain or branched. While the alkyl or alkenyl moiety may have a chain length distribution, it is preferred that 60% by weight or more of alkyl or alkenyl chains have chain lengths within the above-recited range.
Esterification of all the eight hydroxyl groups per sucrose molecule being taken as 100% esterification, the sucrose fatty acid ester is preferably a single compound or a mixture of compounds having a degree of esterification ranging from 2% to 60%. In view of absorption rate and downward wicking properties, the degree of esterification is more preferably 5% to 45%, even more preferably 15% to 35%.

It is preferred for the alkyl glucoside and sucrose fatty acid ester for use as a surface active agent to have an HLB value of 4 to 20. To obtain further improved absorption rate and downward wicking properties, the HLB value is more preferably 9 to 20, even more preferably 12 to 18. For the definition of the HLB value, the formula of W.C. Griffin (see *J. Soc. Cosmetic Chem.* 5 (1954), 249) is applied.

Examples of the hydrophilic cellulosic fiber containing the surface active agent include pulp fiber, cotton, rayon, cuprammonium, Lyocell, and hemp. These fibers may be used either individually or as a mixture of two or more thereof. Using pulp fiber as a main component of hydrophilic cellulosic fiber is preferred for achieving a further increased absorption rate.
While not particularly critical, a part or the whole of the hydrophilic cellulosic fibers containing the surface active agent preferably has a length of 3 to 30 mm and a diameter of 5 to 100 µm in terms of texture or feel and handling properties. It is preferred that the fibers with such length and diameter be present in a proportion of 50% to 100% by weight, more preferably 80% to 100% by weight, based on the total weight of the hydrophilic cellulosic fiber making up a member containing the surface active agent-containing hydrophilic cellulosic fiber (e.g., the cover sheet, absorbent core, or intermediate sheet).
In the cases where the hydrophilic cellulosic fiber has an out-of-round cross-section, the major axis of the cross-section is taken as a diameter. In such cases, it is preferred that an average diameter obtained by measuring at least 10 hydrophilic cellulosic fibers be within the recited range.
The hydrophilic cellulosic fiber may be used in combination with other types of fiber. Nevertheless, the proportion of the hydrophilic cellulosic fiber is preferably at least 50% by weight, more preferably 70% by weight or more, even more preferably 90% by weight or more.

The sanitary napkin 1 of the present embodiment has the surface active agent-containing hydrophilic cellulosic fiber in the absorbent member 4 and/or the intermediate sheet 5. More specifically, each of the fiber aggregate 42, cover sheet 44, and intermediate sheet 5 is made mainly of pulp fiber, and at least part of the pulp fiber in each of them contains the surface active agent. The proportion of the pulp fiber in each of the fiber aggregate 42, cover sheet 44, and intermediate sheet 5 is preferably 50% or more, more preferably 70% or more, even more preferably 90% or more, by weight.

The manner of incorporating the surface active agent into the hydrophilic cellulosic fiber is not particularly restricted. For example, this can be done by applying a solution of the surface active agent (e.g., as diluted with water) either as such or as atomized to the hydrophilic cellulosic fiber or by dipping the fiber in the solution.
The surface active agent-containing hydrophilic cellulosic fiber may have the surface active agent adhered to its surface and/or held inside thereof.

The amount of the surface active agent to be applied is preferably 0.01 to 10 parts, more preferably 0.05 to 5 parts, even more preferably 0.1 to 1 part, most preferably 0.5 to 1 part, by weight per 100 parts by weight of the hydrophilic cellulosic fiber of the absorbent member 4 before application of the surface active agent. These ranges are preferable in view of ensuring the effects as expected and avoiding wastefulness from an overall cost-effectiveness viewpoint. The recited range of the amount is preferred also for the reason that using too much surface active agent can cause blood to greatly reduce in viscosity, which can easily result in rewet. The term "amount to be applied" as used herein means the amount of the surface active agent present in a finished absorbent article.
In cases where the absorbent member 4 contains the surface active agent-containing hydrophilic cellulosic fiber and the superabsorbent polymer 43 as in the napkin 1 of the present embodiment, the proportion of the superabsorbent polymer 43 in the absorbent member 4 is preferably 5 to 300 parts, more preferably 10 to 250 parts, even more preferably 15 to 200 parts, most preferably 20 to 120 parts, by weight per 100 parts by weight of the hydrophilic cellulosic fiber in the absorbent member 4 before application of the surface active agent, in view of amount of rewet.

The sanitary napkin 1 according to the present embodiment, which contains the surface active agent-containing hydrophilic cellulosic fiber in a site other than the skin-contacting surface thereof, has an increased rate of absorption, superior downward wicking properties, and reduced rewet as compared with a sanitary napkin having no surface active agent-containing hydrophilic cellulosic fiber with other conditions being equal. While the reason why has not been fully elucidated as yet, it is believed that the blood discharged on the topsheet 2 undergoes change in viscosity and surface tension on contact with the surface active agent contained in the intermediate sheet 5 and/or the absorbent member 4, whereby it is easily wicked into the absorbent member 4 and is easily absorbed by the superabsorbent polymer. It is unfavorable to use an agent other than the above-described specific surface active agents because such an agent interferes with such changes of blood.

The topsheet 2 and the leak-preventive sheet 3 can be of any material that has been employed in this type of absorbent articles. For example, nonwoven fabrics produced through various processes, perforated resin films, and their composites can be used as the topsheet 2. Moisture-permeable or impermeable thermoplastic resin films, water repellent nonwoven fabrics, and their composites can be used as the leak-preventive sheet 3.
The fiber aggregate 42, cover sheets 44 and 45, and intermediate sheet 5 can be formed of nonwoven fabrics made mainly or solely of cellulosic fiber, fiber webs (not having been consolidated into nonwoven fabrics), or laminates thereof. It is only necessary that at least one member of the fiber aggregate 42, cover sheets 44 and 45, and intermediate sheet 5 contain cellulosic fiber. The other members may be a cellulosic fiber-free nonwoven fabric, fiber web or laminate. When the cover sheets 44 and/or 45 contain the surface active agent-containing hydrophilic cellulosic fiber, it is preferred that the surface active agent-containing hydrophilic cellulosic fiber be present in at least the cover sheet 44 covering the skin-facing side of the absorbent core 41.
It is preferred that the cover sheet 44 have liquid permeability and high wet strength.

The superabsorbent polymer 43 can be of any type that has conventionally been used in this type of absorbent articles. Those capable of absorbing 20 or more times their own weight of liquid and gelling on absorption are preferred. Examples of such superabsorbent polymers include starch, crosslinked carboxymethylated cellulose, copolymers of acrylic acid or its alkali metal salt with other comonomers, polyacrylic acids and alkali metal salts thereof (either partial or complete neutralization products), and polyacrylic acid salt graft polymers. Polyacrylic acid alkali metal salts (either partial or complete neutralization products) are more preferred. These superabsorbent polymers may be used either individually or in combination of two or more thereof.

Fig. 2 is a schematic fragmentary cross-sectional view of a sanitary napkin 1A as a preferred embodiment of the absorbent article according to the second aspect of the present invention. As illustrated in Fig. 2, the sanitary napkin 1A has a skin-contacting surface 20 and includes a liquid permeable topsheet 2 defining the skin-contacting surface 20, a liquid impermeable or repellent leak-preventive sheet 3, an absorbent member 4 interposed between the sheets 2 and 3, and an intermediate sheet 5 interposed between the topsheet 2 and the absorbent member 4. The absorbent member 4 includes an absorbent core 41, a cover sheet 44 covering the skin facing side of the absorbent core 41, and a cover sheet 45 covering the garment facing side of the absorbent core 41. The absorbent core 41 is composed of a fiber aggregate 42 and a superabsorbent polymer 43. The fiber aggregate 42 is made up of pulp fiber.
To avoid redundant description, the napkin 1A will be described primarily with reference to differences from the napkin 1 of the first aspect of the invention. Otherwise, the description about the napkin 1 applies appropriately to the napkin 1A.

The napkin 1A contains synthetic fiber containing at least one surface active agent selected from the group consisting of a sugar alkyl ether and a sugar fatty acid ester in a site other than the skin-contacting surface 20, specifically the intermediate sheet 5.

Examples of the synthetic fiber to be treated with the surface active agent include those made from polyolefin resins such as polyethylene and polypropylene, polyester resins such as polyethylene terephthalate, acrylic resins such as polyacrylic acid and polymethacrylic acid, vinyl resins such as polyvinyl chloride, and polyamide resins such as nylon. Bicomponent conjugate fibers of these resins having a sheath-core configuration or a side-by-side configuration are also useful. These synthetic fibers may be used either individually or as a mixture thereof.

The napkin 1A of the present embodiment contains the surface active agent-containing synthetic fiber in the intermediate sheet 5.
Specifically, the intermediate sheet 5 is a single layer formed of nonwoven fabric containing the surface active agent-containing synthetic fiber.
A fiber layer containing synthetic fiber having been treated with the surface active agent like the nonwoven fabric forming the intermediate sheet 5 is made mainly of synthetic fiber. Specifically, the fiber layer contains more than 50% by weight of synthetic fiber. The proportion of synthetic fiber in the fiber layer as referred to here is the proportion of synthetic fiber based on the total fiber content in the fiber layer including the synthetic fiber having been treated with the surface active agent. The proportion of synthetic fiber is preferably 60% to 100% by weight, more preferably 70% to 100% by weight, and therefore can be 90% by weight or more, or 100% by weight.
Fibers that can be used in combination with the synthetic fiber include hydrophilic cellulosic fibers, such as pulp fiber, cotton, rayon, cuprammonium, Lyocell, and hemp. These hydrophilic cellulosic fibers may be used either individually or as a mixture of two or more thereof.

The manner of treating the synthetic fiber with the surface active agent is not particularly restricted. For example, this can be done by applying a solution of the surface active agent (e.g., as diluted with water) either as such or as atomized to the synthetic fiber or by dipping the fiber in the solution. The synthetic fiber to be treated with the surface active agent may be in the form of fibers or nonwoven fabric.
The surface active agent-treated synthetic fiber may have the surface active agent adhered to its surface and/or held inside thereof.

The sanitary napkin 1A according to the present embodiment, which contains the surface active agent-treated synthetic fiber in the intermediate sheet 5, has an increased rate of absorption, superior downward wicking properties, and reduced rewet as compared with a sanitary napkin having no surface active agent-treated synthetic fiber with other conditions being equal. While the reason why has not been fully elucidated as yet, it is believed that the blood discharged on the topsheet 2 undergoes reduction in viscosity and surface tension on contact with the surface active agent contained in the intermediate sheet 5, whereby it is easily wicked into the absorbent member 4 and is easily absorbed by the superabsorbent polymer.

The amount of the surface active agent to be applied is more than 0.5 parts and not more than 12 parts by weight per 100 parts by weight of the total fiber content of the fiber layer containing the surface active agent-treated synthetic fiber. The amount is preferably 0.6 to 10 parts, more preferably 0.8 to 2 parts, by weight per 100 parts by weight of the fiber content of the fiber layer.
If the amount is 0.5 parts or less, the contact of blood discharged on the topsheet 2 with the surface active agent in the intermediate sheet 5 will be insufficient, causing little change in physical properties of blood. As a result, the above recited effects of the surface active agent, i.e., increasing the absorption rate, improving downward wicking properties, and reducing rewet are not manifested effectively.
Using more than 12 parts of the surface active agent causes a great reduction in blood viscosity, allowing blood to remain in the interfiber spaces in the intermediate sheet 5 or the absorbent member 4 and to cause increased rewet. Furthermore, the surface active agent in the intermediate sheet 5 easily oozes into the topsheet 2 and allows blood to flow on the surface of the topsheet 2 rather than wick inside the absorbent member 4, which is likely to result in leakage.

The topsheet 2, leak-preventive sheet 3, fiber aggregate 42, cover sheets 44 and 45, and superabsorbent polymer 43 of the napkin 1A can be of the same materials as used in the napkin 1.

The intermediate sheet 5 can be formed of the above described nonwoven fabrics, fiber webs before being consolidated into nonwoven fabric, laminates of nonwoven fabrics or webs, or the like. While nonwoven fabrics produced by various processes are usable, air-through nonwoven fabric, hydroentangled nonwoven fabric, and spun-bonded nonwoven fabric are preferred in view of liquid permeability and flexibility.

Fig. 3 is a schematic fragmentary cross-sectional view of a sanitary napkin 1B as a preferred embodiment of the absorbent article according to the third aspect of the present invention. As illustrated in Fig. 3, the sanitary napkin 1B has a skin-contacting surface 20 and includes a liquid permeable topsheet 2 defining the skin-contacting surface 20, a liquid impermeable or repellent leak-preventive sheet 3, an absorbent member 4 interposed between the sheets 2 and 3, and an intermediate sheet 5 interposed between the topsheet 2 and the absorbent member 4. The absorbent member 4 includes an absorbent core 41, a cover sheet 44 covering the skin facing side of the absorbent core 41, and a cover sheet 45 covering the garment facing side of the absorbent core 41. The absorbent core 41 is composed of a fiber aggregate 42 and a superabsorbent polymer 43. The fiber aggregate 42 is made up of pulp fiber.
To avoid redundant description the napkin 1B will be described primarily with reference to differences from the napkin 1 of the first aspect of the invention. Otherwise, the description about the napkin 1 applies appropriately to the napkin 1B.

The napkin 1B contains hydrophilic cellulosic fiber containing at least one surface active agent selected from the group consisting of a sugar alkyl ether and a sugar fatty acid ester in a site other than the skin-contacting surface 20. Specifically, the surface active agent is present in the absorbent core 41 composed of the fiber aggregate 42 and the superabsorbent polymer 43.

As stated, the napkin 1B of the present embodiment contains the specific surface active agent in the absorbent core 41 constituting the absorbent member 4.
In the present embodiment the surface active agent is preferably present on the surface and/or in the inside of the superabsorbent polymer or adjacent to the superabsorbent polymer.

In the cases where the surface active agent is present on the surface and/or in the inside of the superabsorbent polymer, the surface active agent preferably covers a part of, or the whole of, the surface of the polymer particles.
The location of the surface active agent can be confirmed by analyzing a cross-section of the polymer particles by time of flight secondary ion mass spectrometry (TOF-SIMS).

The method of locating the surface active agent on the surface and/or inside of the superabsorbent polymer particles is not particularly restricted.
For example, superabsorbent polymer particles having the surface active agent on their surface and in the inside thereof are preferably obtained by spraying a solution of the surface active agent (e.g., as diluted with water) to superabsorbent polymer particles followed by drying.
Superabsorbent polymer particles having the surface active agent in the inside thereof are preferably obtained by washing the thus obtained polymer having the surface active agent on the surface and inside the particles.
Superabsorbent polymer particles having the surface active agent on their surface are preferably obtained by spraying water to superabsorbent polymer particles, mixing the wetted polymer particles with the surface active agent of solid form, followed by drying.

The superabsorbent polymer having the surface active agent on the surface and/or in the inside thereof is preferably distributed in a fiber aggregate to make the absorbent core 41 together with the fiber aggregate.
Configurations of the superabsorbent polymer being distributed in a fiber aggregate include a structure in which the polymer particles are three-dimensionally distributed between fibers constituting the fiber aggregate and a structure in which a layer of the polymer particles is sandwiched between two layers of the fiber aggregate.
The fiber aggregate constituting the absorbent core 41 may be either a web of fiber before being consolidated into nonwoven fabric, nonwoven fabric, or a laminate of such a web and nonwoven fabric.

Now, the expression "the surface active agent is present adjacent to the superabsorbent polymer" as used herein is intended to mean the state in which the surface active agent, either alone or in the form of a surface active agent-containing material, is located near the superabsorbent polymer such that the surface active agent comes into contact with blood almost simultaneously with, or slightly earlier than, the superabsorbent polymer. In order for the surface active agent to come into contact with blood slightly earlier than the superabsorbent polymer, it is preferred that the surface active agent, either alone or in the form of a surface active agent-containing material, be located nearer to the skin-contacting surface than is the superabsorbent polymer within the absorbent core 41. It is more preferred that the surface active agent or the surface active agent-containing material be present in contact with the superabsorbent polymer.

Methods for locating the surface active agent adjacent to the superabsorbent polymer preferably include, but are not limited to, a method in which the surface active agent of solid form and the superabsorbent polymer particles are mixed and spread between two layers of a fiber aggregate and a method in which the superabsorbent polymer particles are spread on a fiber aggregate coated with the surface active agent.

In the present embodiment, the constituent fiber of the fiber aggregate making up the absorbent core 41 together with the superabsorbent polymer can be selected from various types of fibers conventionally used in absorbent members with no particular limitation, including hydrophilic cellulosic fibers such as pulp and synthetic fibers. The fiber aggregate may be a mixture of hydrophilic cellulosic fiber such as pulp and synthetic fiber or a laminate of a hydrophilic cellulosic fiber layer and a synthetic fiber layer.
Examples of the hydrophilic cellulosic fiber include pulp fiber, cotton, rayon, cuprammonium, Lyocell, and hemp. Examples of the synthetic fiber include those made from polyolefin resins such as polyethylene and polypropylene, polyester resins such as polyethylene terephthalate, acrylic resins such as polyacrylic acid and polymethacrylic acid, vinyl resins such as polyvinyl chloride, and polyamide resins such as nylon. Bicomponent conjugate fibers of these resins having a sheath-core configuration or a side-by-side configuration are also useful.

The amount of the surface active agent to be applied is 1.5 to 15 parts by weight, preferably 2.0 to 13 parts by weight, more preferably 3.0 to 10 parts by weight, per 100 parts by weight of the superabsorbent polymer present in the absorbent article (namely, the absorbent core in the present embodiment).
These ranges are preferable in view of ensuring the effects as expected and avoiding wastefulness from an overall cost-effectiveness viewpoint The term "amount to be applied" as used herein means the amount of the surface active agent present in a fmished absorbent article. The amount of the solvent, etc. of a surface active agent solution, etc. is not counted. When the surface active agent is present on the surface and/or in the inside of the superabsorbent polymer particles, the term "100 parts by weight of the superabsorbent polymer" as referred to in reciting the amount of the surface active agent is the weight exclusive of the surface active agent.
When the absorbent core 41 contains a superabsorbent polymer as with the case of the napkin 1B, the amount of the superabsorbent polymer 43 (in the case of the superabsorbent polymer having the surface active agent on the surface and/or in the inside thereof, the amount of the superabsorbent polymer includes the amount of the surface active agent) in the absorbent core 41 is preferably 5% to 300% by weight, more preferably 15% to 200% by weight, even more preferably 20% to 120% by weight, based on the weight of the fiber aggregate.

The sanitary napkin 1B according to the present embodiment, which contains 1.5 to 15 parts by weight of the specific surface active agent per 100 parts by weight of the superabsorbent polymer, has an increased rate of absorption, superior downward wicking properties, and reduced rewet. While the reason why has not been fully elucidated as yet, it is believed that the blood discharged on the topsheet 2 undergoes change in viscosity and surface tension on contact with the surface active agent present on the surface or near the surface of the superabsorbent polymer, whereby blood is easily wicked into the interfiber spaces of the absorbent core and is easily absorbed by the superabsorbent polymer.

The topsheet 2, leak-preventive sheet 3, and superabsorbent polymer 43 of the napkin 1B can be of any materials conventionally employed in this type of articles, including those recited above for use in the napkin 1.
The cover sheets 44 and 45 and intermediate sheet 5 can be formed of sheeting of fibrous materials, including nonwoven fabrics or webs of hydrophilic fibers such as the above described hydrophilic cellulosic fiber or hydrophilized synthetic fiber, laminates of nonwoven fabrics or webs, paper, and the like. It is preferred for the cover sheet 44 to have high wet strength as well as liquid permeability.

While the present invention has been described with reference to its preferred embodiment, the absorbent article of the invention is not limited to the above embodiment, and various modifications can be made therein within the spirit and scope of the invention. The following is a few examples of such modifications.
In the above-described embodiment, the fiber aggregate 42, cover sheet 44, and intermediate sheet 5 of the napkin 1 are all made mainly of pulp fiber, and the pulp fiber in at least part of each of them contains the surface active agent. This configuration may be changed such that only one of the pulp fiber of the fiber aggregate 42, that of the cover sheet 44, and that of the intermediate sheet 5 contains the surface active agent.
Even when the surface active agent is present in only one of the fiber aggregate 42, the cover sheet 44, and the intermediate sheet 5, there are exerted improving effects on absorption rate, downward wicking properties, and reduced rewet.

While in the napkin 1A the surface active agent-treated synthetic fiber is contained in the nonwoven fabric forming the intermediate sheet 5, the fiber layer containing the surface active agent-treated synthetic fiber may be the fiber layer (e.g., nonwoven fiber) forming the cover sheet 44 or any but the uppermost, skin-contacting-side-defining layer of a multilayered laminate nonwoven fabric used as a topsheet 2.

The intermediate sheet 5 and the cover sheets 44 and 45 may be dispensed with where unnecessary. For example, whereas each sanitary napkin described above has all of the intermediate sheet 5 and the cover sheets 44 and 45, one or more of these sheets may be dispensed.

While in the above embodiments the superabsorbent polymer 43 in the absorbent core 41 is uniformly distributed throughout the fiber aggregate 42 as illustrated in Figs. 1 through 3, it may be localized in the thickness direction of the fiber aggregate 42, e.g., near the topsheet, the leak-preventive sheet or the center of the thickness. The superabsorbent polymer 43 may be sandwiched in between layers of a multi-layered fiber aggregate 42.

The absorbent article of the present invention includes a sanitary napkin as exemplified by the above-described embodiment, a panty liner, an incontinence pad, and a disposable diaper.

The fiber layer that can be used in the absorbent article of the invention may have the surface active agent uniformly distributed in the planar direction thereof or may include a region where the surface active agent is present and a region where the surface active agent is absent arranged mixedly in the planar direction thereof. As used herein, the terminology "planar direction of the fiber layer" means a direction perpendicular to the thickness direction of the fiber layer.
For example, the fiber layer used in the absorbent article of the first aspect of the invention may have the surface active agent uniformly distributed in the planar direction thereof or may include a region having the surface active agent and a region having no surface active agent arranged mixedly in the planar direction thereof.

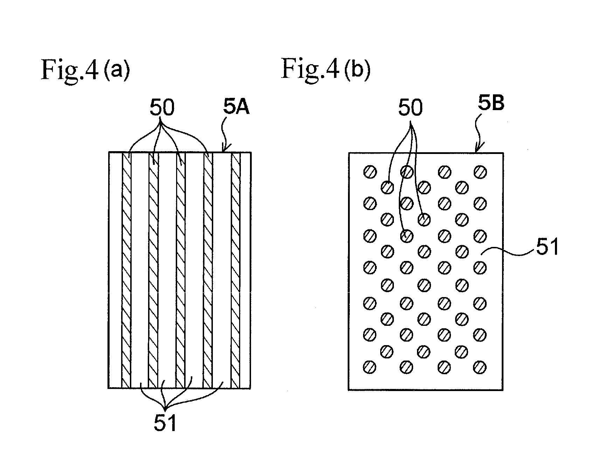

Figs. 4(a) and 4(b) represent fiber layers 5A and 5B (e.g., a nonwoven fabric used as an intermediate sheet 5), in which regions 50 having the surface active agent applied and regions 51 having no surface active agent applied are discretely arranged in a certain pattern. The area ratio of the regions 50 to the regions 51 (the regions 50 : the regions 51) may range, for example, from 10:90 to 80:20.
In the case when the surface active agent is patternwise arranged in a planar direction of the fiber layer, the surface active agent can be applied in any manner, for example, with a brush or through a spray nozzle.

The fiber layer 5A illustrated in Fig. 4(a) has the regions 50 (having the surface active agent) arranged in stripes. In more detail, the fiber layer 5A has the surface active agent applied in stripes in one planar direction thereof to form regions 50 spaced apart by regions 51 with no surface active agent.
Each region 50 may have a width of, for example, 2 to 20 mm, and each region 51 may have a width of, for example, 1 to 10 mm.
Mixed arrangement of the regions 50 having the surface active agent and the regions 51 having no surface active agent in a planar direction of the fiber layer provides the following advantage. The surface active agent in the regions 50 modifies a discharged body fluid into a fluid that is wicked into the absorbent member more easily and is absorbed by the superabsorbent polymer more easily. On the other hand, the existence of the regions 51 having no surface active agent and therefore remaining highly hydrophilic secure a good balance of improvements in absorption rate and low rewet.
A body fluid easily flows in the direction of the stripe-shaped regions 50 and hardly flows in the direction perpendicular thereto. Accordingly, the fiber layer 5A can be disposed in an absorbent article with the direction of the regions 50 coinciding with the longitudinal direction of the absorbent article (i.e., the front-to-rear direction of a wearer) to provide an improved protection against side leakage.

The fiber layer 5B illustrated in Fig. 4(b) has the regions 50 (having the surface active agent) discretely arranged in dots in a staggered pattern. Each region 50 may have a circular shape as illustrated or any other shape, e.g., a cube, a rectangle, a diamond or a heart. Each region 50 can have an area of, for example, 5 to 100 mm². Every adjacent regions 50 may be spaced by a center-to-center distance of, e.g., 3 to 10 mm. This pattern of arrangement of the regions 50 and 51 produces the same effects on absorption rate and rewet as with the pattern of Fig. 4(a).

### [Example]

The present invention will now be illustrated in greater detail with reference to Examples, but it should be understood that the invention is not construed as being limited thereto. Unless otherwise noted, all the percents and parts are given by weight.

### Examples and Comparative Examples of First Aspect of Invention

### Example 1

A 1% aqueous solution of decyl glucoside (Mydol 10, a surface active agent available from Kao Corp.) was sprayed to fluff pulp (NB416, available from Weyerhaeuser Company; average length: 3 mm; diameter (major axis): 10-30 µm) to adhere 0.6 parts of decyl glucoside per 100 parts of the pulp fiber. The pulp fiber was dried by blowing hot air at 80°C and 3% RH in a dryer for 2 hours. The dried pulp fiber was shaped into an absorbent core measuring 75 mm in width, 200 mm in length, and 2 mm in thickness. Absorbent paper (cover sheet) made of pulp fiber and having a grammage of 16 g/m² was wrapped around the skin-facing side and the garment-facing side of the absorbent core to make an absorbent member weighing 200 g/m². The resulting absorbent member had 0.5 parts of the surface active agent adhered to 100 parts of the hydrophilic cellulosic fiber thereof. The absorbent member was assembled into a sanitary napkin in combination with the same topsheet, intermediate sheet, and backsheet (corresponding to the leak-preventive sheet) as used in a commercially available sanitary napkin (Laurier Super Slim Guard - Day Use, available from Kao Corp.).

### EXAMPLES 2 TO 22

The surface active agent shown in Table 1 below was applied to100 parts of the same fluff pulp as used in Example 1 in the amount shown in Table 1 in the same manner as in Example 1. The pulp fiber as dried was mixed up with 30 parts of a superabsorbent polymer (Aqualic CA-4 available from Nippon Shokubai Co., Ltd.) and shaped into a fluff fiber/superabsorbent polymer-mixed sheet as an absorbent core measuring 75 mm in width and 200 mm in length and weighing 168 g/m². Absorbent paper (cover sheet) made of pulp fiber and having a grammage of 16 g/m² was wrapped around the skin-facing side and the garment-facing side of the absorbent core to make an absorbent member weighing 200 g/m². The resulting absorbent member was assembled into a sanitary napkin in the same manner as in Example 1.

### EXAMPLE 23

Ninety parts of the same fluff pulp as used in Example 1 and 10 parts of rayon fiber (length: 10-20 mm; diameter (major axis): 10-24 µm) were mixed. The same surface active agent solution as used in Example 1 (1% aqueous solution of decyl glucoside) was sprayed to the mixed fiber to adhere 0.6 parts of decyl glucoside per 100 parts of the mixed fiber and dried in the same manner as in Example 1. A sanitary napkin was obtained in the same manner as in Examples 2 to 22, except for using the resulting surface active agent-containing mixed fiber to make the absorbent core.

### EXAMPLE 24

A fluff pulp/superabsorbent polymer-mixed sheet as an absorbent core was made in the same manner as in Example 2, except that the surface active agent was not applied to the pulp fiber.
The same surface active agent solution as used in Example 1 (1% aqueous solution of decyl glucoside) was sprayed onto absorbent paper (cover sheet) made of pulp fiber and having a grammage of 16 g/m² to adhere 0.6 parts of decyl glucoside per 100 parts of the total hydrophilic cellulosic fiber (pulp fiber) inclusive of that in the absorbent core. The absorbent paper was dried by blowing hot air at 80°C and 3% RH in a dryer for 2 hours.
The absorbent core was wrapped in the surface active agent-containing absorbent paper (cover sheet) to obtain an absorbent member and assembled into a sanitary napkin in the same manner as in Example 1.

### EXAMPLE 25

A pulp fiber/superabsorbent polymer-mixed sheet as an absorbent core weighing 170 g/m² was made in the same manner as in Example 2, except that the surface active agent was not applied to the pulp fiber. The resulting absorbent core was employed as an absorbent member.
The same surface active agent solution as used in Example 1 (1% aqueous solution of decyl glucoside) was sprayed onto a pulp sheet made of crosslinked pulp fiber (High Bulk Additive, from Weyerhaeuser Company) and having a grammage of 30 g/m² to adhere 2.4 parts of decyl glucoside per 100 parts of the crosslinked pulp fiber of the pulp sheet. The surface active agent solution was sprayed uniformly in the planar directions of the pulp sheet (directions perpendicular to the thickness direction). The pulp sheet was dried by blowing hot air at 80°C and 3% RH in a dryer for 2 hours and cut to size (75 mm x 200 mm).
The resulting pulp sheet was overlaid on the skin facing side of the absorbent member as an intermediate sheet. The stack of the absorbent member and the pulp sheet was assembled into a sanitary napkin in the same manner as in Example 1, using the same topsheet and the backsheet as in Example 1.
The amount of the decyl glucoside in the sanitary napkin, although not directly adhered to the absorbent member, was 0.6 parts per 100 parts of the hydrophilic cellulosic fiber of the absorbent member and 0.5 parts per 100 parts of the total hydrophilic cellulosic fiber content; the sum of the crosslinked pulp fiber of the intermediate sheet and the pulp fiber of the absorbent member.

### EXAMPLE 26

A 2% aqueous solution of the same surface active agent (decyl glucoside) as used in Example 1 was applied with a brush to the same pulp sheet as used in Example 25 in stripes of 2 mm width (corresponding to regions 50 in Fig. 4(a)) spaced by a distance of 3 mm (corresponding to regions 51 in Fig. 4(a)). The area ratio of the regions 50 to the regions 51 (the regions 50 : the regions 51) was 3:2. The amount of the surface active agent applied was 2.4 parts per 100 parts of the crosslinked pulp fiber of the pulp sheet.
A sanitary napkin was assembled in the same manner as in Example 25, except for using the resulting pulp sheet as an intermediate sheet.
The amount of the surface active agent in the sanitary napkin, although not directly adhered to the absorbent member, was 0.6 parts per 100 parts of the hydrophilic cellulosic fiber of the absorbent member and 0.5 parts per 100 parts of the total hydrophilic cellulosic fiber content; the sum of the crosslinked pulp fiber of the intermediate sheet and the pulp fiber of the absorbent member.

### EXAMPLE 27

A 2% aqueous solution of the same surface active agent (decyl glucoside) as used in Example 1 was applied with a brush to the same pulp sheet as used in Example 25 in circular dots arranged in a staggered pattern as shown in Fig. 4(b). Each of the circular regions 50 having the surface active agent applied had an area of 78 mm², and the center-to-center distance between every adjacent circular regions 50 was 15 mm. The area ratio of the regions 50 to the region 51 (region with no surface active agent applied) was 78:63 (the regions 50 : the region 51).
A sanitary napkin was assembled in the same manner as in Example 25, except for using the resulting pulp sheet as an intermediate sheet.
The amount of the surface active agent in the sanitary napkin, although not directly adhered to the absorbent member, was 0.6 parts per 100 parts of the hydrophilic cellulosic fiber of the absorbent member and 0.5 parts per 100 parts of the total hydrophilic cellulosic fiber content; the sum of the crosslinked pulp fiber of the intermediate sheet and the pulp fiber of the absorbent member.

### COMPARATIVE EXAMPLE 1

A sanitary napkin was made in the same manner as in Example 1, except that the surface active agent solution was not sprayed.

### COMPARATIVE EXAMPLE 2

A sanitary napkin was made in the same manner as in Example 2, except that the surface active agent solution was not sprayed.

### COMPARATIVE EXAMPLE 3

The same surface active agent solution (1% aqueous solution of decyl glucoside) as used in Example 1 was sprayed to the same topsheet as used in Example 2 to adhere 8 parts of decyl glucoside per 100 parts of the topsheet. A pulp fiber/superabsorbent polymer-mixed sheet as an absorbent core was made in the same manner as in Example 2, except that the surface active agent was not applied to the pulp fiber. Otherwise in the same manner as in Example 2, a sanitary napkin was assembled.

### COMPARATIVE EXAMPLES 4 TO 6

A sanitary napkin was prepared in the same manner as in Example 2, except that the surface active agent of the kind and amount shown in Table 1 was applied to the fluff pulp.

### Evaluation of absorption rate, downward wicking properties, and amount of rewet

The sanitary napkins obtained in Examples 1 to 27 and Comparative Examples 1 to 6 were evaluated in terms of absorption rate, downward wicking properties, and amount of rewet in accordance with the following test methods and rated based on an A to E scale according to the following standards. The results obtained are shown in Table 1.
The sanitary napkin was placed horizontally. An acrylic resin plate having a 1 cm diameter pour hole and weights were put thereon to apply a load of 5.0 g/cm² (490 Pa) to the napkin. Six grams of defibrinated horse blood (available from Nippon Biotest Lab.) was poured through the pour hole in about 1 second, and the time required for the blood to be completely absorbed was measured to calculate the absorption rate. After the pouring, the napkin was left to stand for 3 minutes with the acrylic resin plate and weights thereon.
The resin plate and weights were removed, and the liquid spread area was measured using the following method to evaluate downward wicking properties.
Subsequently, a stack of 10 absorbent paper sheets (5 commercially available 2-ply tissues; 7 cm x 10 cm; grammage: 30 g/m² each) was placed on the skin contacting side of the napkin, and weights were put thereon to apply a pressure of 6.6 x 10³ Pa for 2 minutes. The placement of tissues and the weights had to be done within 4 minutes after the pouring. The stack of 10 absorbent paper sheets was removed and weighed. The amount of the blood absorbed by the tissues was obtained as a difference from the weight before absorption, which was taken as the amount of rewet.

### Measurement of liquid spread area

The liquid spread of the absorbent core was scanned into a computer using a light source (Sunlight SL-230K2, supplied by LPL Co., Ltd.), a stand (Copy Stand CS-5, from LPL Co., Ltd.), a lens (Nikkol 24 mm/F2.8D, from Nikon Corp.), a CCD camera (HV-37, from Hitachi Electronics, Ltd., to which the lens was attached with an F mount), and a video board (Spectra 3200, from Canopus Co., Ltd.). The display size and the pixel size should be correlated to each other by calibration to allow for area measurement.
Then, liquid spread in the absorbent core, the measuring object, was scanned to obtain an image data thereof. The image data were binarized using image analyzing software NewQube ver. 4.21 (from Nexus) to obtain the liquid spread area.

Standards of rating:
(1) Absorption rate (Abs. rate)
   A: 0.2 g/sec or higher
   B: 0.17 g/sec or higher and lower than 0.2 g/sec
   C: 0.14 g/sec or higher and lower than 0.17 g/sec
   D: 0.1 g/sec or higher and lower than 0.14 g/sec
   E: lower than 0.1 g/sec

(2) Downward wicking properties
   A: liquid spread area of less than 27 cm²
   B: liquid spread area of 27 cm² or more and less than 30 cm²
   C: liquid spread area of 30 cm² or more and less than 32 cm²
   D: liquid spread area of 32 cm² or more and less than 35 cm²
   E: liquid spread area of 35 cm² or more

(3) Amount of rewet (rewet amt.)
   A: less than 0.9 g
   B: 0.9 g or more and less than 1.0 g
   C: 1.0 g or more and less than 1.1 g
   D: 1.1 g or more and less than 1.2 g
   E: 1.2 g or more

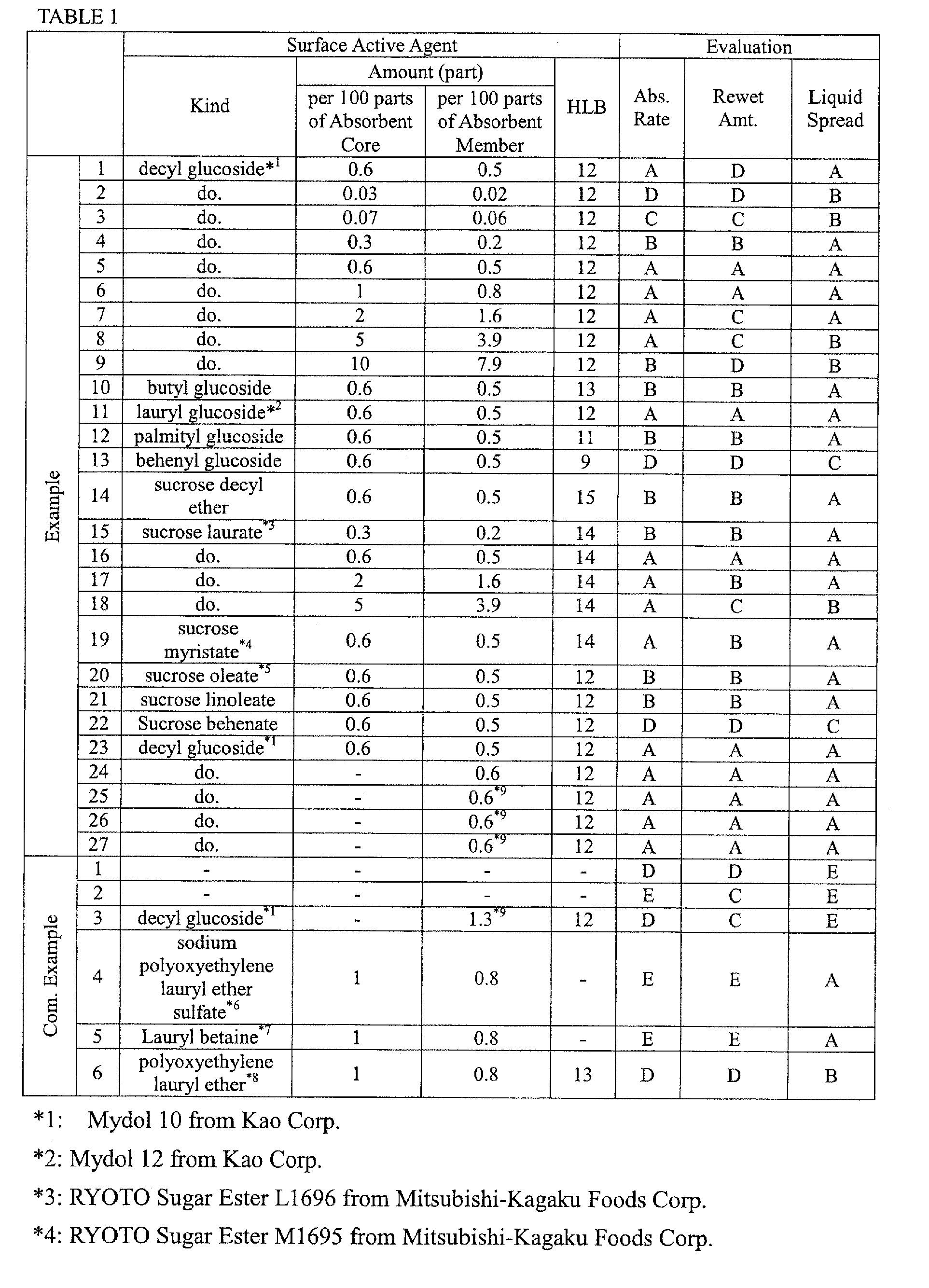

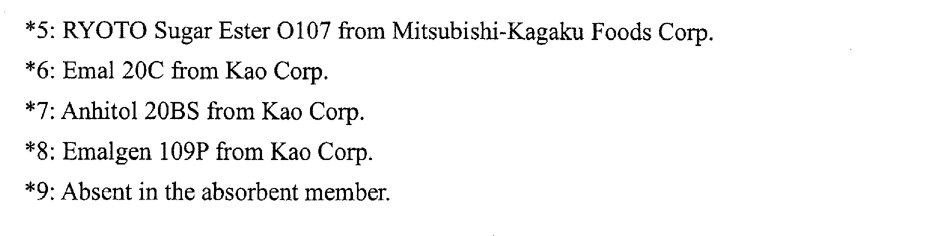

As can be seen from Table 1, the sanitary napkins of the present invention are satisfactory in all of absorption rate, reduced rewet, and downward wicking performance. They show particularly great improvements in downward wicking performance over the comparative napkins.

Examples and Comparative Examples of Second Aspect of Invention

### EXAMPLE 28

An intermediate sheet was taken out of a commercially available sanitary napkin (Laurier Super Slim Guard - Day Use, from Kao Corp.). The intermediate sheet was air-through nonwoven fabric made out of sheath/core conjugate fiber having a polyethylene sheath and a polyethylene terephthalate core.
A 1% aqueous solution of decyl glucoside (Mydol 10, a surface active agent available from Kao Corp.) was sprayed onto the entire area of the intermediate sheet uniformly in the planar directions of the intermediate sheet (directions perpendicular to the thickness direction) to adhere 0.6 parts of decyl glucoside per 100 parts of the intermediate sheet. The intermediate sheet was then dried by blowing hot air at 80°C and 3% RH in a dryer for 2 hours.
The dried intermediate sheet was assembled with the same topsheet, leak-preventive sheet, and absorbent member as used in the napkin, Laurier Super Slim Guard - Day Use, to make a sanitary napkin having the same structure as Laurier Super Slim Guard - Day Use.

### EXAMPLES 29 TO 44

A sanitary napkin was prepared in the same manner as in Example 28, except that the surface active agent of the kind and amount (per 100 parts of the intermediate sheet) shown in Table 2 below was applied to the same intermediate sheet as used in Example 28 in the same manner as in Example 28.

### EXAMPLE 45

A 2% aqueous solution of the same surface active agent as used in Example 28 (decyl glucoside) was applied with a brush to the same intermediate sheet as used in Example 28 in stripes of 3 mm width (corresponding to regions 50 in Fig. 4(a)) spaced by a distance of 2 mm (corresponding to regions 51 in Fig. 4(a)). The area ratio of the regions 50 to the regions 51 (the regions 50 : the regions 51) was 3:2.
A sanitary napkin was assembled in the same manner as in Example 28, except for using the resulting intermediate sheet. The amount of the surface active agent applied was 0.6 parts per 100 parts of the intermediate sheet.

### EXAMPLE 46

A 2% aqueous solution of the same surface active agent as used in Example 28 (decyl glucoside) was applied with a brush to the same intermediate sheet as used in Example 28 in circular dots arranged in a staggered pattern as shown in Fig. 4(b). Each circular region 50 had a circular shape having an area of 78 mm². Every adjacent regions 50 were spaced by a center-to-center distance of 5 mm. The area ratio of the regions 50 to the regions 51 (the regions 50 : the regions 51) was 74:66.
A sanitary napkin was assembled in the same manner as in Example 28, except for using the resulting intermediate sheet. The amount of the surface active agent applied was 0.6 parts per 100 parts of the intermediate sheet.

### COMPARATIVE EXAMPLE 7

A sanitary napkin was prepared in the same manner as in Example 28, except that the surface active agent was not applied to the intermediate sheet.

### COMPARATIVE EXAMPLES 8 AND 9

A sanitary napkin was prepared in the same manner as in Example 28, except that the surface active agent of the kind and amount shown in Table 2 was applied in the same manner as in Example 28.

The sanitary napkins obtained in Examples 28 to 46 and Comparative Examples 7 to 9 were evaluated in terms of absorption rate, downward wicking properties, and amount of rewet in the same manner as described above. The results obtained are shown in Table 2.

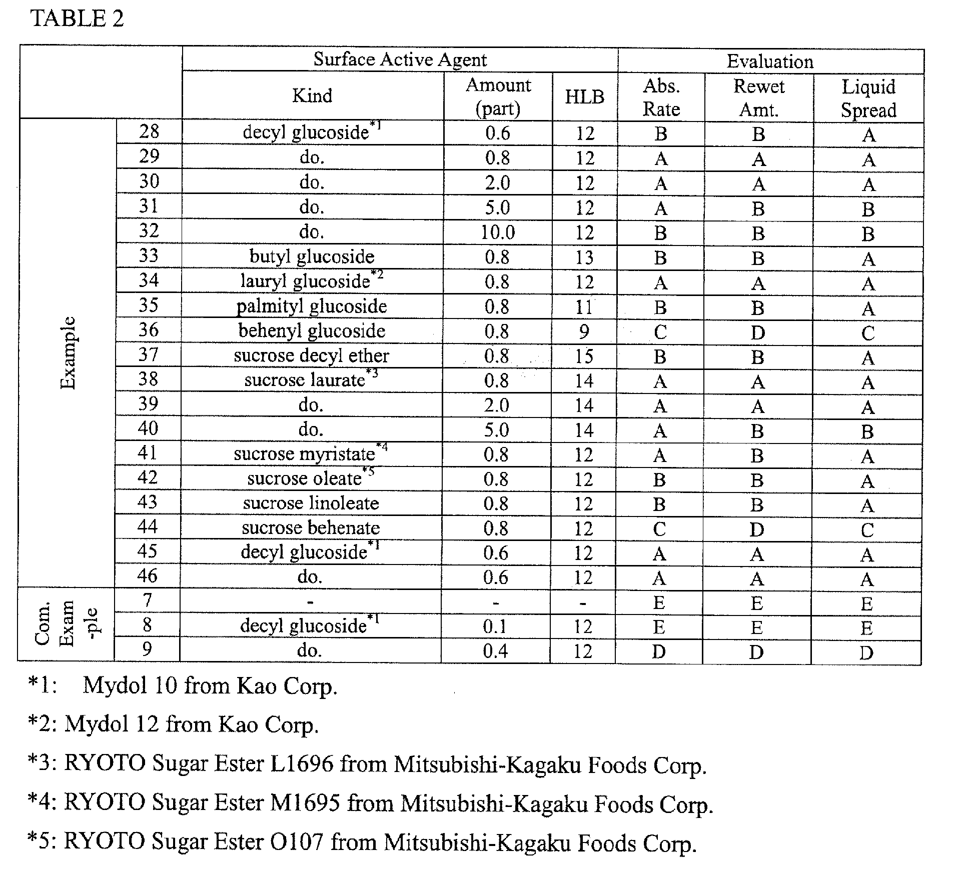

As can be seen from Table 2, the sanitary napkins of the present invention are satisfactory in all of absorption rate, reduced rewet, and downward wicking performance. They show particularly great improvements in downward wicking performance over the comparative napkins.

### Examples and Comparative Examples of Third Aspect of Invention

### EXAMPLE 47

A superabsorbent polymer (Aqualic CA-4 available from Nippon Shokubai Co., Ltd.) was spread thin on a horizontal place, and a 1% aqueous solution of decyl glucoside (Mydol 10, a surface active agent available from Kao Corp.) was sprayed thereto. The polymer was dried by blowing hot air at 80°C and 3% RH in a dryer overnight to obtain a superabsorbent polymer having the surface active agent on its surface and in its inside. The location of the surface active agent was confirmed by analyzing a cross-section of the polymer particles by TOF-SIMS.
Two sheets of fluff pulp having 100 g/m² in weight (NB416, available from Weyerhaeuser Company) each measuring 75 mm in width, 200 mm in length, and 1 mm in thickness were prepared. The surface active agent-treated polymer was sandwiched between the two sheets in an amount of 60 parts per 100 parts of the total fluff pulp to prepare an absorbent core. A wet-processed pulp sheet having a grammage of 20 g/m² (NB416, available from Weyerhaeuser Company) was wrapped around the skin facing side and the garment facing side of the absorbent core to make an absorbent member.
The absorbent member was assembled into a sanitary napkin in combination with the same topsheet, intermediate sheet, and backsheet (leak-preventive sheet) as used in a commercially available sanitary napkin (Laurier Super Slim Guard - Day Use, from Kao Corp.).
The resulting sanitary napkin contained 2.0 parts of the surface active agent per 100 parts of the superabsorbent polymer. The surface active agent content per 100 parts of the superabsorbent polymer was calculated from the weight change between before and after the treatment with the surface active agent.

### EXAMPLES 48 TO 63

A sanitary napkin was prepared in the same manner as in Example 47, except that the surface active agent of the kind and amount shown in Table 3 below was applied in the same manner as in Example 47.

### EXAMPLE 64

Decyl glucoside (Mydol 10 from Kao Corp.) was freeze dried to obtain a solid surface active agent.
Two parts of the solid surface active agent was mixed with 100 parts of a superabsorbent polymer (Aqualic CA-4 available from Nippon Shokubai Co., Ltd.). The resulting mixture was spread between a pair of fluff pulp sheets (NB416, available from Weyerhaeuser Company) each measuring 75 mm in width, 200 mm in length, 1 mm in thickness, and 100 g/m² in weight to make an absorbent core containing 60 parts of the superabsorbent polymer per 100 parts of the total fluff pulp fiber. The absorbent core was covered on its skin facing and garment facing sides with a wet-processed pulp sheet having a grammage of 20 g/m² (NB416, from Weyerhaeuser Company) to make an absorbent member.
The absorbent member was assembled into a sanitary napkin in combination with the same topsheet, intermediate sheet, and backsheet (leak-preventive sheet) as used in a commercially available sanitary napkin (Laurier Super Slim Guard - Day Use, available from Kao Corp.).

### EXAMPLES 65 AND 66

A sanitary napkin was prepared in the same manner as in Example 64, except for changing the kind and amount of the surface active agent as shown in Table 3.

### COMPARATIVE EXAMPLE 10

A sanitary napkin was prepared in the same manner as in Example 47, except that the superabsorbent polymer was not sprayed with Mydol 10.

### COMPARATIVE EXAMPLE 11

A sanitary napkin was prepared in the same manner as in Example 47, except for changing the amount of the surface active agent as shown in Table 3.

The sanitary napkins obtained in Examples 47 to 66 and Comparative Examples 10 and 11 were evaluated in terms of absorption rate, downward wicking properties, and amount of rewet in the same manner as described above. The results obtained are shown in Table 3.

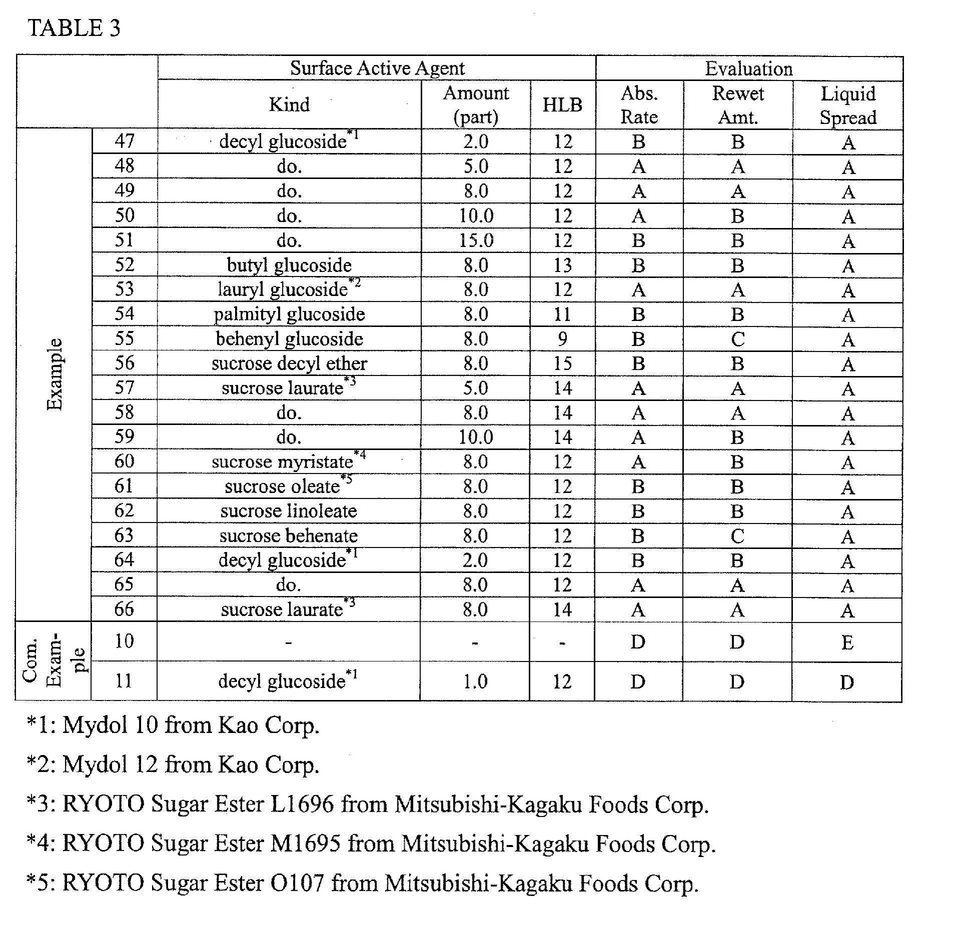

As can be seen from Table 3, the sanitary napkins of the present invention are satisfactory in all of absorption rate, reduced rewet, and downward wicking performance.

### Industrial Applicability

The sanitary napkins of the present invention have an increased rate of absorption, superior downward wicking properties, and reduced rewet.

## Claims

1. An absorbent article comprising hydrophilic cellulosic fiber,
the hydrophilic cellulosic fiber containing at least one surface active agent selected from the group consisting of a sugar alkyl ether and a sugar fatty acid ester,
the hydrophilic cellulosic fiber containing the surface active agent being present in a site other than the skin-contacting surface.

2. The absorbent article according to claim 1, wherein the surface active agent has an HLB value of 9 to 20.

3. The absorbent article according to claim 1 or 2, wherein the hydrophilic cellulosic fiber containing the surface active agent is fibers having a length of 3 to 30 mm and a diameter of 5 to 100 µm.

4. The absorbent article according to any one of claims 1 to 3, wherein the surface active agent is present in an amount of 0.1 to 1 part by weight per 100 parts by weight of the hydrophilic cellulosic fiber of an absorbent member.

5. The absorbent article according to any one of claims 1 to 4, comprising a liquid permeable topsheet defining a skin-contacting surface of the absorbent article, a liquid impermeable or repellent leak-preventive sheet, and an absorbent member disposed between the topsheet and the leak-preventive sheet,
the absorbent member comprising the hydrophilic cellulosic fiber containing the surface active agent.

6. The absorbent article according to claim 5, wherein the absorbent member further comprises a superabsorbent polymer, the proportion of the superabsorbent polymer in the absorbent member being 20 to 120 parts by weight per 100 parts by weight of the hydrophilic cellulosic fiber.

7. The absorbent article according to claim 5 or 6, wherein the absorbent member comprises an absorbent core and a cover sheet wrapping the absorbent core, the absorbent core containing the hydrophilic cellulosic fiber containing the surface active agent.

8. The absorbent article according to claim 5 or 6, wherein the absorbent member comprises an absorbent core and a cover sheet wrapping the absorbent core, the cover sheet containing the hydrophilic cellulosic fiber containing the surface active agent.

9. The absorbent article according to any one of claims 1 to 8, comprising a liquid permeable topsheet defining a skin-contacting surface of the absorbent article, an absorbent member, and an intermediate sheet disposed between the topsheet and the absorbent member, the intermediate sheet comprising the hydrophilic cellulosic fiber containing the surface active agent.

10. The absorbent article according to claim 1, comprising a fiber layer comprising the hydrophilic cellulosic fiber containing the surface active agent, the fiber layer having regions with the surface active agent applied and regions with no surface active agent applied arranged mixedly in a planar direction thereof.

11. An absorbent article comprising a fiber layer containing more than 50% by weight of synthetic fiber, the synthetic fiber comprising synthetic fiber treated with at least one surface active agent selected from the group consisting of a sugar alkyl ether and a sugar fatty acid ester,
the surface active agent being present in an amount more than 0.5 parts and not more than 12 parts by weight per 100 parts by weight of the total fiber content of the fiber layer.

12. The absorbent article according to claim 11, further comprising a liquid permeable topsheet defining the skin-contacting surface of the absorbent article, an absorbent member, and an intermediate sheet interposed between the topsheet and the absorbent member, the intermediate sheet comprising the fiber layer.

13. The absorbent article according to claim 12, wherein the absorbent member contains a superabsorbent polymer.

14. The absorbent article according to any one of claims 11 to 13, wherein the fiber layer has regions where the surface active agent is present and regions where the surface active agent is absent arranged mixedly in a planar direction thereof.

15. An absorbent article containing a superabsorbent polymer and 1.5 to 15 parts by weight of at least one surface active agent selected from the group consisting of a sugar alkyl ether and a sugar fatty acid ester per 100 parts by weight of the superabsorbent polymer.

16. The absorbent article according to claim 15, wherein the surface active agent is present on the surface and/or in the inside of the superabsorbent polymer.

17. The absorbent article according to claim 15, wherein the surface active agent is present adjacent to the superabsorbent polymer.
